(19) Europäisches Patentamt

European Patent Office

Office européen des brevets

(11) **EP 1 302 532 B1**

(12) **EUROPÄISCHE PATENTSCHRIFT**

(45) Veröffentlichungstag und Bekanntmachung des Hinweises auf die Patenterteilung:
**02.03.2005 Patentblatt 2005/09**

(51) Int Cl.[7]: **C12N 1/14**
// C12R1:645

(21) Anmeldenummer: **02021859.0**

(22) Anmeldetag: **30.09.2002**

(54) **Verfahren zur Erhöhung der Wachstumsrate von Zellen in statischen Flüssigmedien**

Method for increasing the growth rate of cells in static liquid culture

Méthode pour élever le taux de prolifération de cellules dans une culture liquide statique

(84) Benannte Vertragsstaaten:
**AT BE BG CH CY CZ DE DK EE ES FI FR GB GR IE IT LI LU MC NL PT SE SK TR**

(30) Priorität: **11.10.2001 DE 10150311**

(43) Veröffentlichungstag der Anmeldung:
**16.04.2003 Patentblatt 2003/16**

(73) Patentinhaber: **Bayer CropScience AG**
**40789 Monheim (DE)**

(72) Erfinder:
  • **Aichinger, Christian, Dr.**
    **50939 Köln (DE)**
  • **Schreier, Peter, Prof. Dr.**
    **50674 Köln (DE)**
  • **Schöneberg, Dana**
    **42327 Wuppertal (DE)**

(56) Entgegenhaltungen:
   **EP-A- 0 164 888** **WO-A-01/73099**

  • **DATABASE WPI [Online] DERWENT PUBLICATIONS LTD., LONDON, GB; Ungarisches Patent HU30776, 28. März 1984 (1984-03-28) "Binder for viscous culture medium - comprises microcrystalline cellulose and agar-agar." retrieved from WPI Database accession no. 1984-122671 XP002224284**
  • **GARCA-OCHOA F ET AL: "Xanthan gum: production, recovery, and properties" BIOTECHNOLOGY ADVANCES, ELSEVIER PUBLISHING, BARKING, GB, Bd. 18, Nr. 7, 1. November 2000 (2000-11-01), Seiten 549-579, XP004317586 ISSN: 0734-9750**

**Beschreibung**

Die vorliegende Erfindung betrifft ein Verfahren zur Erhöhung der Wachstumsrate von Zellen und der Expression von Genen in diesen Zellen in statischen Flüssigmedien durch den Einsatz von Xanthan im Medium sowie die Verwendung von Gelbildnern wie Xanthan in solchen Verfahren.

Für eine Reihe von Mikroorganismen wie Bakterien oder Pilze wie z.B. *S. cerevisiae, B. cinerea, S. tritici, M. grisea, P. infestans* oder *R. solani* sind seit langem geeignete Wachstumsbedingungen für die Verwendung artifizieller Nährmedien beschrieben. Auch für Säuger- oder Insektenzellkulturen sowie Pflanzenzellkulturen sind solche artifiziellen Medien bekannt. Diese Medien werden z.B. auch in Hochdurchsatz-Verfahren eingesetzt, die auf der Verwendung von Zellkulturen beruhen. So ist z.B. *S. cerevisiae* erfolgreich in auf Zellen basierenden Assays in HTS-Experimenten verwendet worden. Es ist jedoch ebenfalls bekannt, dass für eine große Anzahl verschiedener Zelltypen wie z.B. von Pilzen Wachstum oder Keimung auf Plattenmedien möglich ist, jedoch die Anzucht in Flüssigmedien entweder nicht möglich ist oder sehr aufwendig und damit unbefriedigend ist (z. B. Rostpilze, Oomyceten). Eine dritte Gruppe von Pilzen, sogenannte obligat biotrophe, kann nicht auf künstlichen Medien gehalten werden. Es handelt sich dabei um Pilze, die eine langfristige Nahrungs-Beziehung mit lebenden Zellen ihres Wirts eingehen. Die Anzucht von Zellen in Flüssigmedien erfordert prinzipiell, dass die Medien kontinuierlich durchmischt, d. h. geschüttelt werden, um eine Versorgung der Zellen mit Nährstoffen zu gewährleisten. Die meisten Zellen, z.B. Pilzzellen, sind deshalb für HTS-Verfahren schlecht oder nicht geeignet, in denen die Flüssigmedien nicht geschüttelt werden können, also statisch sind. HTS-Verfahren erfordern in der Regel aber Wachstum oder zumindest das Überleben der Zellen. Darüber hinaus ist in aller Regel die Expression eines Reportergens notwendig. Der Einsatz einer hohen Zelldichte ist hier kein Ausweg, da eine zu hohe Zelldichte Messungen der optischen Dichte oder der Fluoreszenz unmöglich macht. Diese Problematik soll am folgenden Beispiel weiter verdeutlicht werden.

Neben den erwähnten Hefepilzen gibt es weitere Pilze, die sowohl in Festmedien als auch in Flüssigmedien hervorragende Wachstumseigenschaften besitzen. Ein Vertreter dieser Gruppe ist der phytopathogene Basidiomycet *Ustilago maydis. U. maydis* wird als dimorpher Pilz beschrieben. Im haploiden Stadium zeigt er hefeähnliches Wachstum (Sporidie) mit einer Generationszeit von etwa zwei Stunden in Vollmedium. Die zweite Wuchsform, das dikaryotische Filament proliferiert hingegen nicht auf artifiziellen Medien. Deshalb sind die haploiden Sporidien die hauptsächliche Kulturform von *U. maydis.* Um optimales Wachstum zu erreichen ist es nötig, die Kulturen unter Schütteln zu ziehen.

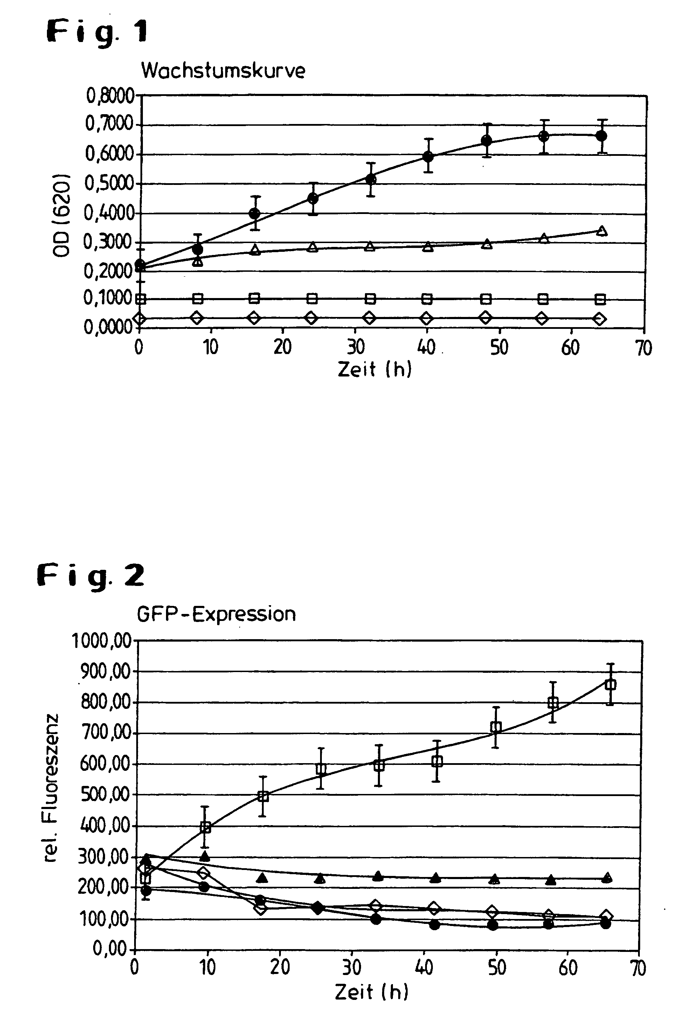

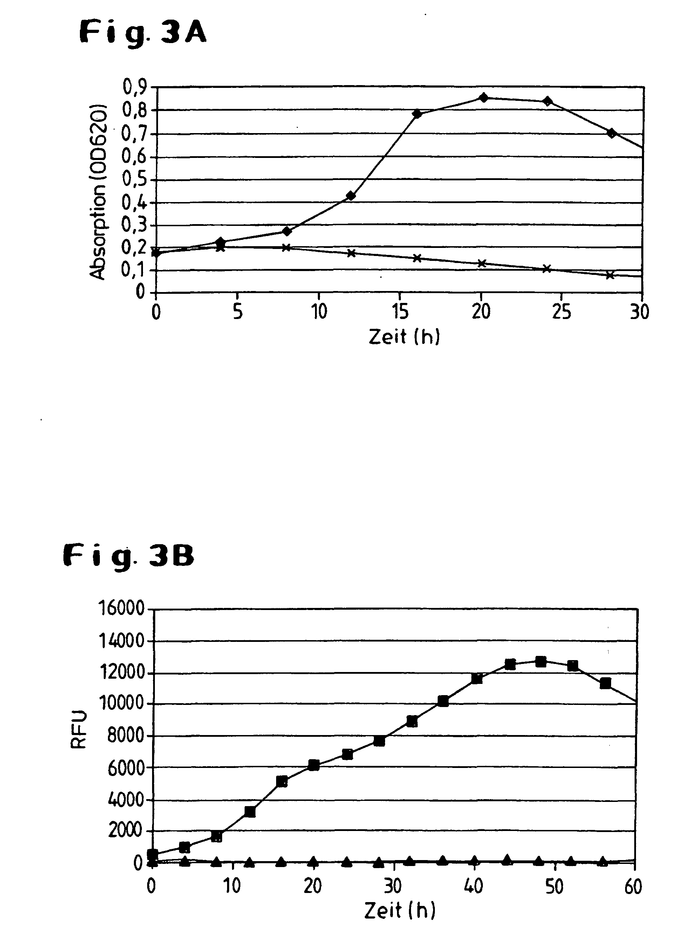

Wird eine *U. maydis* Kultur dagegen in eine statische, nicht konstant geschüttelte bzw. durchmischte Lösung, wie z.B. in eine 384-well-MTP (MTP ≙ Mikrotiterplatte), überführt, so ist keine Zunahme der optischen Dichte zu beobachten (Abbildung 1), es findet also kein Wachstum statt. Es ist deshalb nicht möglich, Pilzzellen wie z.B. *U. maydis* Zellen in solchen statischen Flüssigmedien anzuziehen und zu verwenden. Dies ist vor allem dann problematisch, wenn bei einer Versuchsanordnung aufgrund der spezifischen Eigenschaften eines Targets, eines spezifischen Enzymtests oder der verwendeten Messmethode nur ein *in vivo* Tests möglich ist oder gewünscht wird. Dazu gehören unter anderem Targets wie Membranproteine oder auch Rezeptoren sowie Polypeptide, deren Aufreinigung aus einer Zelle schwierig ist oder die mit einem Aktivitätsverlust des Polypeptids verbunden ist.

Zudem ist bei Zellen, die ein konstitutiv exprimiertes Reportergen tragen, keine oder nur eine geringfügige Steigerung der Expression zu beobachten (Abbildung 2). Dies macht die Verwendung der oben genannten Pilzzellen in statischen Flüssigmedien bislang unmöglich.

Die genannten Probleme wirken sich somit negativ bei der Hochdurchsatz-Suche nach neuen, im Falle des genannten Beispiels fungiziden Wirkstoffen aus, die neuen Wirkstoffklassen angehören und/oder neue Wirkorte haben. Wirkorte, also Targets bzw. Polypeptide oder auch DNAs oder RNAs, die nicht in der üblichen Weise in einem bakteriellen Wirt überproduziert, aufgereinigt und in *in vitro*-HTS-Assays eingesetzt werden können, sind deshalb nur sehr schwer zugänglich. Somit entfällt das Screening eine Reihe interessanter Targets aufgrund technischer Probleme. Häufig ist jedoch gerade die Nutzung der Organismen, z. B. Pilze, in denen diese Targets identifiziert werden konnten, geeignet, um das betreffend Polypeptid zu exprimieren und unmittelbar *in vivo* zu testen. Die beschriebenen Probleme haben jedoch bislang nicht erlaubt, bestimmt Zellen bzw. Organismen wie z.B. die vorstehend genannten Pilze dafür zu verwenden.

Im Rahmen der vorliegenden Erfindung wurde nun festgestellt, dass das mangelnde Wachstum der Zellen darauf zurückzuführen ist, dass sie auf den Boden der Behältnisse, z.B. der Mikrotiterplatten, absinken, wodurch die notwendige Versorgung mit Sauerstoff und Nährstoffen nicht mehr gewährleistet ist. Die Sauerstoffsättigung des Mediums in der Kavität ist von der Diffussionsgeschwindikeit und von der Austauschoberfläche mit der Luft abhängig, die in den beschriebenen statischen Flüssigmedien nur sehr gering sind. Ein weiteres Problem kann die so genannte Kontaktinhibition von Zellwachstum sein, wobei Zellen, sobald sie einander berühren, die Proliferation einstellen (z. B. Osteoblasten).

Analoge Probleme wie vorstehend beschrieben ergeben sich bei allen Zellen, die in Flüssigkulturen absinken und nur dann in einem signifkanten Maße wachsen können, wenn eine konstante Durchmischung des Mediums z.B.

im Schüttler erfolgt, und die Versorgung der Zellen mit Nährstoffen und Sauerstoff dadurch gewährleistet wird. Davon können Pflanzenzellen, Säuger- oder Insektenzellen betroffen sein. Auch Zellen von *S. cerevisiae*, die an sich in der Lage sind, auch unter anaeroben Bedingungen zu wachsen, zeigen bei Sauerstoffzufuhr eine deutlich bessere Vermehrungsrate. Der Bergriff "Zellen", wie er hierin verwendet wird, umfasst deshalb alle hier genannten Zell- und Zellkulturtypen, also z.B. Pilzzellen, Pflanzenzellen, Säugerzellen und Insektenzellen.

Aufgabe der vorliegenden Erfindung war es, ein Verfahren zur Verfügung zu stellen, mit dem ein Wachstum von Zellen in unbewegten Flüssigmedien gewährleistet wird, das also die Verwendung der Zellen in den genannten statischen Medien ermöglicht.

**Das Wachstum von *U. maydis* in 384-well-MTPs wird durch die Zugabe eines Xanthans ermöglicht**

Es wurde nun überraschenderweise gefunden, dass durch die Zugabe geeigneter Konzentrationen eines Xanthans in das Flüssigmedium das Wachstum von Zellen wie z.B. Pilzzellen ermöglicht wird, was anhand der Zunahme der optischen Dichte und der erhöhten Expression eines Reportergens nachgewiesen werden kann.

Mit Hilfe der vorliegenden Erfindung wird nunmehr z.B. die Sauerstoff- und Nährstoffversorgung für die Zellen verbessert, indem dem Flüssigmedium ein Xanthan zugegeben wird, das die Zellen in Suspension hält.

Dabei ist die vorliegende Erfindung nicht auf bestimmte Medien beschränkt, soweit das betreffende Medium für die verwendete Zellkultur sowie den Verwendungszweck der Zellkultur geeignet ist. Das heißt, das Medium muss z.B. für die Messmethode zur Bestimmung des Wachstums der Zellen oder der Aktivität eines bestimmten Genprodukts geeignet sein, die bei der gewählte Zellkultur bzw. dem gewählten Reportergen verwendet werden soll. So muss bei der Verwendung von Green Fluorescent Protein (GFP) als Reportergen in einer Zellkultur gewährleistet sein, dass das Medium keine Eigenfluoreszenz aufweist. So wäre z.B. das sogenannte Potato Dextrose Medium (Tsukada et al., 1988) für Fluoreszenzmessungen basierend auf eGFP nicht geeignet, da dieses Medium im Wellenlängenbereich von eGFP mit der Messung interferiert. Auch die Messung des Wachstum der Zellen, in der Regel die Messung der optischen Dichte, verlangt ein Medium, das die OD-Messung nicht beeinflusst (wenig geeignet sind hier z.B. Medien die Blut enthalten).

Ein Beispiel für ein solches Xanthan ist das so genannte Kelzan®, ein Xanthan-Polysaccharid (Xanthan-Gummi). Durch die Anwesenheit des Xanthans wird erreicht, dass Zellen, z.B. Pilzzellen, gleichmäßig in der Kavität verteilt bleiben und so mit genügend Sauerstoff und Nährstoffen versorgt und nicht von einer Kontaktinhibition betroffen werden.

Die optimale Verdoppelungszeit im Schüttelkolben liegt z.B. für *U. maydis*-Zellen bei nur 2,5 h. Für den beispielhaft verwendeten *U. maydis* Stamm Um518 konnte innerhalb von 65 Stunden keine Verdopplung in statischem Flüssigmedium ohne Xanthan beobachtet werden. Die optische Dichte nahm im untersuchten Zeitraum nur um etwa 75 % zu. In Medium mit Xanthan wurde jedoch bereits nach etwa 21 Stunden die Zellzahl verdoppelt. Die optische Dichte von *U. maydis* verdreifacht sich innerhalb von 65 Stunden, wenn Xanthan zum Medium zugegeben wird. Die Wachstums- und Proliferationsbedingungen für die Pilzzellen werden damit deutlich verbessert, wodurch die Verwendung solcher Zellen in Verfahren ermöglicht wird, die ansonsten nur die Nutzung statischer Kulturen erlauben.

**Der Einfluss von Xanthanen auf die Expression eines Genprodukts am Beispiel der Expression von GFP in *U. maydis* Reporterstämmen in 384 well-MTPs**

Der Wachstumstest zeigt bereits den positiven Einfluss eines Xanthans auf die Wachstumsrate von *U. maydis* Zellen. Neben dem Wachstum wurde wie vorstehend beschrieben bisher auch gleichzeitig die Expression von Polypeptiden verhindert, was die Nutzung z.B. von Pilzzellen in *in vivo* Testsystemen, z.B. in HTS-Assays, unmöglich macht. Häufig muss erst eine verstärkte Produktion (Überproduktion) des interessierenden, zu testenden Polypeptids erfolgen, um Aktivitätstests mit einem ausreichenden Signal zu ermöglichen. Bei *in vivo*-Verfahren ist es jedoch auch häufig nötig, die Aktivität bzw. den Aktivitätsverlust eines Polypeptids oder eines Polypeptidkomplexes mit Hilfe eines Reporters zu verfolgen. Ein typisches Beispiel für einen Reporter ist das "Green Fluorescent Protein" (GFP), dessen Expression anhand seiner charakteristischen Fluoreszenzeigenschaften gemessen werden kann.

Im Rahmen der vorliegenden Erfindung wurde deshalb untersucht, ob sich das vorstehend beschriebene verstärkte Wachstum auch auf die Expression von Genprodukten auswirkt, bzw. ob das verstärkte Wachstum genügt, um eine ausreichende Expression für Aktivitätstests oder HTS zu gewährleisten. Dies sollte exemplarisch anhand der Expression eines Reportergens untersucht werden. Für *U. maydis* eignet sich dafür besonders das oben genannte GFP, weil es für seine Aktivität keine weiteren exo- oder endogenen Faktoren benötigt.

Für die Bestimmung der GFP Fluoreszenz wurde der *U. maydis* Stamm UMA3 verwendet, der eGFP (Clontech: "enhanced green fluorescent protein") unter dem konstitutiven otef-Promotor (siehe Beispiel 3) exprimiert. Um die Hintergrundfluoreszenz zu bestimmen, wurde die GFP-Fluoreszenz mit der Fluoreszenz im Wildtypstamm Um518 verglichen. Die Fluoreszenz in den Stämmen wurde jeweils mit und ohne Xanthan im Medium bestimmt (Minimalmedium). Als weitere Kontrollen wurde das verwendete Medium allein (ohne Zellen) mit und ohne Xanthan in die Untersuchung mit einbezogen. Bestimmt wurde die auf GFP beruhende Fluoreszenz in mindestens 36 Kavitäten bei einer Anregungswellenlänge von 485 nm (10 nm Bandbreite) und einer Emissionswellenlänge von 510 nm (10 nm Bandbreite) (Beispiel 4, Abbildung 2).

Dabei konnte beobachtet werden, dass es zu einer deutlichen Zunahme der Fluoreszenz beim Stamm UMA3 in solchen Kavitäten kommt, die Xanthan als Gelbildner im Medium enthielten. Im Gegensatz dazu war in UMA3 Stämmen, die ohne Xanthan im Medium inkubiert wurden, keine Zunahme der GFP-Fluoreszenz zu erkennen. Es wurde sogar eine leichte Abnahme der Fluoreszenz beobachtet. Gleiches gilt für den Stamm Um518, der das GFP Reportergen nicht enthält. Die Reduktion der GFP-Fluoreszenz ist demnach nicht vom GFP-Reportergen abhängig sondern muss andere Ursachen haben. Die Medienkontrollen zeigten ebenfalls keine signifikanten Änderungen in der Fluoreszenz (nicht gezeigt).

Es konnte damit im Rahmen der vorliegenden Erfindung weiter gezeigt werden, dass der Zusatz eines Xanthans zu Medien zu einer signifikanten Erhöhung der Proteinexpression in den Zellen führt. Darin spiegelt sich vermutlich die oben gezeigte Verbesserung der Wachstumsrate mit diesem Zusatz wieder. Die Xanthane schaffen also Bedingungen in Zellkulturen wie z.B. von Pilzzellen wie *U. maydis*, die eine signifikante Erhöhung der Zellteilungsrate ermöglichen. Diese geht mit einer ebenfalls signifikant höheren Proteinexpression einher.

Damit ermöglicht die vorliegende Erfindung die Durchführung von auf Zellen basierenden *in vivo* Tests in Verfahren wie dem HTS oder UHTS, die bislang solchen Verfahren nur eingeschränkt zugänglich waren.

Gegenstand der vorliegenden Erfindung ist damit ein Verfahren zur Erhöhung der Wachstumsrate von Zellen, bevorzugt von Pilzzellen, in statischen Flüssigmedien, dadurch gekennzeichnet, das das Medium ein Xanthan enthält. Besonders bevorzugt eignet sich das erfindungsgemäße Verfahren in Zellkulturen von *U. maydis*.

Gegenstand der vorliegenden Erfindung ist ebenfalls ein Verfahren zur Erhöhung der Expression eines Gens in Zellkulturen, bevorzugt in Pilzzellkulturen, in statischen Flüssigmedien, dadurch gekennzeichnet, dass das Medium ein Xanthan enthält. Besonders bevorzugt eignet sich das erfindungsgemäße Verfahren in Zellkulturen von *U*. *maydis*.

Gegenstand der vorliegenden Erfindung ist ebenfalls die Verwendung von Xanthanen in statischen Flüssigmedien zur Erhöhung der Wachstumsrate von Zellkulturen, bevorzugt von Pilzzellkulturen, und zur Erhöhung der Expression eines Gens in Zellen, bevorzugt in Pilzzellen. Besonders bevorzugt kann Xanthan erfindungsgemäß in Zellkulturen von *U. maydis* verwendet werden.

Gegenstand der vorliegenden Erfindung ist ebenfalls die Verwendung von Xanthanen in statischen Flüssigmedien in *in vivo* HTS- oder UHTS-Assays mit Pilzzellkulturen. Besonders bevorzugt kann Xanthan erfindungsgemäß in HTS- oder UHTS-Assays mit Zellkulturen von *U. maydis* verwendet werden.

## Verbesserung der Wachstumsrate von Zellkulturen

Wie bereits vorstehend ausgeführt, sind z.B. für bestimmte Pilze bzw. Pilzzellen bereits Verfahren entwickelt worden, die deren Nutzung in HTS-Assays bzw. in statischen Flüssigmedien gestatten. Für eine Reihe von Pilzzellen ergeben sich bei den beschriebenen Verfahren keine Wachstumsprobleme, so dass diese bevorzugt verwendet wurden und werden.

Pilze, bei denen sich die vorstehend genannten Probleme ergeben, sind z.B. solche Pilze, die in statischen Flüssigmedien zur Sedimentation und Aggregation neigen und deshalb zum einen nicht mehr den gängigen Messmethoden zugänglich sind, und zum anderen durch die mangelnde Sauerstoff und Nährstoffversorgung nicht mehr wachsen oder absterben. Ein Beispiel dafür ist der pflanzenpathogene Basidiomycet *Ustilago maydis*. Dieser Pilz wird bereits seit geraumer Zeit erforscht und ist deshalb auch im Rahmen der wichtigen Targetforschung, also der Suche nach Angriffspunkten für neue Fungizide, von besonderer Bedeutung. Sogenannte Targets, die in *U. maydis* identifiziert und erforscht werden konnten und in Zukunft noch gefunden werden, die jedoch für *in vitro* Assays aufgrund einer problematischen Aufreinigung oder ihrer spezifischen Aktivität (Membranproteine, Rezeptoren etc.) bislang nicht zugänglich waren, konnten bislang jedoch nur begrenzt und unter beträchtlichem Aufwand zur Suche nach neuen Wirkstoffen genutzt werden. Man war vielmehr gezwungen, die homologen Gene in anderen, geeigneteren Organismen zu identifizieren und/oder zu exprimieren und eventuell aufzureinigen.

Diese Problematik ist jedoch nicht auf *U. maydis* beschränkt. Andere Zellen und andere Pilze können deshalb in analoger Weise durch den Einsatz von Xanthanen für die Verwendung in statischen Flüssigmedien und damit in HTS- oder UHTS-Assays zugänglich gemacht werden. Dazu gehört auch der Pilz *S. cerevisiae*, der zwar bereits aufgrund seiner Fähigkeit anaerob zu leben auch in statischen Flüssigmedien verwendet werden konnte, dessen Wachstum und Fähigkeit zur Expression von Polypeptiden durch das erfindungsgemäße Verfahren weiter verbessert werden kann.

Es gibt eine Reihe von Verbindungen, häufig Biopolymere, aber auch synthetische Polymere oder Mischungen derselben, die in der Lage sind, Gele bzw. wässrige Gele zu bilden und die als Stabilisatoren, Emulsionsvermittler, Verdickungsmittel, oder zur Hydrokolloidbildung oder Texturierung verschiedener Medien dienen. Diese "Gelbildner" kommen in der Lebensmittelverarbeitung aber auch für industrielle Zwecke, z.B. bei Ölbohrungen, zum Einsatz. Bekannt ist auch deren Verwendung für Formulierungen, z. B. bei der Saatgutbehandlung, wo verschiedene Polymere ein wässriges Gel bilden, das die Keimung von Bioherbiziden wie *Alternaria cassiae*, einem Pilz, befördert (Shabana et al., 1997).

Besonders interessant sind natürlich vorkommende Polysaccharidverbindungen wie Xanthane, deren Verwendung vor allem in Lebensmitteln gängige Praxis ist. Aufgrund ihrer spezifischen Eigenschaften, die am Beispiel des Kelzans nachstehend näher erläutert werden, eignen sich die genannten Polymere gut für die vorstehend beschriebenen erfindungsgemäßen Verfahren.

Xanthane werden z.B. von *Xanthomonas campestris* sowie einigen verwandten Mikroorganismenarten gebildet. Das Polysaccharid wird aus dem Medium gewonnen, indem es mit Isopropanol in Gegenwart von KCl gefällt wird. Xanthan ist ein Heteroglykan, das D-Glucose, D-Mannose, D-Glucuronsäure, Acetat und Pyruvat im molaren Verhältnis 28:30:20:17:5,1-6,3 enthält. Das Molekulargewicht variiert von $2x10^6$ bis $12x10^6$, je nach Herstellbedingungen und verwendetem Stamm. Xanthan ist in kaltem und heißem Wasser gut löslich, wobei die sich bildenden Helices ein dreidimensionales Netzwerk ausbilden, welches hohe Viskositäten mit sich bringt. In hochviskosen Lösungen zeigt es daher pseudoplastisches Verhalten. Die Viskosität ist dabei weitgehend unabhängig von der Temperatur. Lösungen, Emulsionen und Gele besitzen in Gegenwart von Xanthan eine hohe Gefrier-Tau-Stabilität. Xanthan wird zur Druckstabilisierung und zur Stabilisierung von Emulsionen etherischer Öle in Getränken eingesetzt. Wegen seiner großen thermischen Stabilität wird es auch als Dickungsmittel bei Konserven eingesetzt. Bei Stärkegelen verbessert ein Zusatz von Xanthan die Gefrier-Tau-Stabilität. Im Ruhezustand erreicht man eine hohe Stabilität, während bei einer Scherbeanspruchung der Viskositätsabfall ein leichtes Fließen bewirkt. Xanthane eignen sich besonders gut zur Verwendung in den erfindungsgemäßen Verfahren.

Ein Beispiel für ein Xanthan ist das oben erwähnte Kelzan® (CAS#: 11138-66-2). Kelzan® ist bis zu einer Temperatur von 130°C wirksam. Das Polymer ist leicht anionisch, weshalb der Mischvorgang mit einer Lösung, die kationisch ist, vorsichtig erfolgen muss. Kelzan® ist in besonderem Maße dazu geeignet in den vorstehend beschriebenen erfindungsgemäßen Verfahren verwendet zu werden.

Die Konzentration der Xanthane kann variiert und in einfachen Versuchen an das vorliegende Medium, die verwendeten Zellen und den Verwendungszweck angepasst werden, solange gewährleistet ist, dass die Zellen einerseits nicht oder ausreichend langsam absinken, und andererseits die Lösung noch pipettiert werden kann und etwaige Messungen nicht gestört werden. Wichtig ist auch, dass die Konzentration so angepasst wird, dass die beim Vermischen von Medium mit Zellen eventuell entstehenden Luftbläschen aufsteigen und entweichen können, da diese ansonsten Messungen entscheidend stören können.

Die Konzentration der Xanthane (z.B. Kelzan®) kann über einen relativ großen Bereich variiert werden. Als besonders geeignet hat sich eine Konzentration von 0,08 bis 0,3 Gew.-% erwiesen. Optimale Ergebnisse wurden bei einer Konzentration zwischen 0,09 und 0,12 Gew.-%, insbesondere 0,1 Gew.-% erzielt.

Der Temperaturbereich, in dem die Xanthane verwendet werden können, ist in aller Regel groß und kann den vorstehenden Ausführungen oder der Fachliteratur entnommen werden. Die für Zellkulturen interessanten Temperaturbereiche von 18 bis 38°C stellen für die Xanthane kein Problem dar.

Für die Xanthane, insbesondere Kelzan®, wurden sehr gute Ergebnisse bei einer Temperatur von 16 bis 37°C, besonders bei einer Temperatur von 22 bis 34°C erreicht, wobei insbesondere bei 28°C bis 30°C gute Ergebnisse erzielt wurden. Optimale Ergebnisse wurden bei einer Temperatur von 30°C erreicht.

Die gelbildende Wirkung der Xanthane ist in der Regel unabhängig vom vorgelegten Volumen. Xanthane (z. B. das Kelzan®) wurden z.B. erfolgreich in Zellkulturen in 384-well MTPs (100µl) und in Zellkulturen in Erlenmeierkolben (800 ml) eingesetzt.

Neben dem Kelzan® gibt es noch eine Reihe anderer Xanthane, die ähnliche Eigenschaften aufweisen, und deshalb in gleicher Weise im erfindungsgemäßen Verfahren verwendet werden können.

## Beispiele

## Beispiel 1

### Wachstumsbedingungen für *U. maydis*

*U. maydis* Zellen wurden in PD-Medium bei 28°C bis zu einer $OD_{600}$ von 0,6 bis 1,0 kultiviert. Danach wurden sie 10 min bei 3200 rpm abzentrifugiert, mit Minimalmedium gewaschen und anschließend so in Minimalmedium bzw. Minimalmedium mit 0,1% Kelzan® resuspendiert, dass die Kultur eine $OD_{600}$ von 0,3 zeigt. Dazu kann folgende Berechnung herangezogen werden:

$$V(x) = V(y) * OD_{600}(y) / OD_{600}(x)$$

V    Volumen

x:    Pilzkultur in PD-Medium aus Übernachtkultur

y:    Pilzkultur in Minimalmedium bzw. Minimalmedium mit 0,1 % Kelzan®, die in den MTP-Test eingesetzt wird.

Für den Wachstumstest wurden je 50 µl Zellsuspension mit einem Multidrop Pipettierer in 384-well-MTPs (transparent, Firma Greiner) pipettiert. Die Absorption wurde für den Wachstumstest bei einer Wellenlänge von 620 nm bestimmt. Die Platten wurden über drei Tage bei Raumtemperatur inkubiert. Zum Zeitpunkt null und danach alle vier Stunden wurde die optische Dichte in einem Tecan plus "reader" bestimmt (Abbildung 1).

### Verwendete *U. maydis*-Stämme

Für die durchgeführten Versuche wurden die *Ustilago maydis*-Stämme Um518 (*a2b2*) und UMA3 (*a2b2* pCA123) verwendet.

| Verwendete Medien | |
|---|---|
| PD-Medium | 2,4 % (w/v) Potato Dextrose Broth |
| Minimalmedium (MM) | 0,3 % (w/v) $KNO_3$<br>6,25 % (v/v) Salzlösung nach Holliday, 1974<br>1,0 % (w/v) Glucose |
| Salzlösung (nach Holliday, 1974) | 16,0 % (w/v) $KH_2PO_4$<br>4,0% (w/v) $Na_2SO_4$<br>8,0 % (w/v) KCl<br>4,08 % (w/v) $MgSO_4*7H_2O$<br>1,32 % (w/v) $CaCl_2*2H_2O$<br>8,0 % (v/v) Traceelements nach Holliday, 1974 |
| Traceelements (nach Holliday, 1974) | 0,06 % (w/v) $H_3BO_3$<br>0,14 % (w/v) $MnCl*4H_2O$<br>0,4 % (w/v) $ZnCl_2$<br>0,4 % (w/v) $Na_2MoO_4*2H_2O$<br>0,1 % (w/v) $FeCl_3*6H_2O$<br>0,04 % (w/v) $CuSO_4*5H_2O$ |

### Beispiel 2

### Wachstumstest mit *U. maydis* Zellen und Kelzan®

Mit einem Wachstumstest (M&M) wurde der Einfluss von 0,1 % Kelzan® im Medium auf die Wachstumsrate der *U. maydis*-Zellen untersucht. Dazu wurde der *U. maydis* Wildtyp-Stamm Um518 in Minimalmedium mit 0,1 % Kelzan® und ohne Kelzan® in MT-Platten inkubiert. Zur Kontrolle wurden parallel dazu die optische Dichte des Minimalmediums allein bzw. des Minimalmediums mit 0,1 % Kelzan® bestimmt. Die Absorptionsmessung erfolgte bei einer Wellenlänge von 620 nm alle 4h in 36 unabhängigen Kavitäten. Die Messungen liefen über drei Tage (65 Stunden), wobei alle 4 h eine Messung erfolgte.

### Beispiel 3

### Plasmidkonstruktion von pCA123 zur Messung der Exprimierung von GFP in Flüssigmedium

Aus dem Plasmid potef-SG (Spellig et al., 1996) wurde der otef Promotor als 890 bp langes PvuII/NcoI-Fragment isoliert und in den PvuII/NcoI geschnittenen Vektor pTEF-SG (Spellig et al., 1996) ligiert. Im resultierenden Plasmid wurde das SGFP Gen durch eine Restriktion mit NcoI/NotI herausgeschnitten und durch das NcoI/NotI geschnittene EGFP-Allel aus pEGFP-NI (Fa. Clontech) ersetzt. Das resultierende Plasmid heißt pCA123.

**Beispiel 4**

**Bestimmung der Expression des Reportergens GFP**

Je 50μl Zellsuspension der zu testenden Stämme wurden wie oben beschrieben in 384-well-MTPs (schwarz, Firma Greiner) überführt. Die relative Fluoreszenz durch das exprimierte GFP wurde fluorimetrisch bei einer Anregungswellenlänge von 485 nm (Bandbreite 10 nm) und einer Emissionswellenlänge von 510 nm (Bandbreite 10 nm) sowie einem Gainfaktor von 70 bei 3 Blitzen/ Messung in einem Tecan plus "reader" bestimmt. Die Messungen erfolgten zum Zeitpunkt null und danach alle 4h (Abbildung 2).

**Beschreibung der Abbildungen**

**Abbildung 1**

**Kelzan®-abhängiges Wachstum von *U. maydis* Zellen in 384-well-MTPs.** Das Wachstum ist als Zunahme der optischen Dichte bei einer Wellenlänge von 620 nm abhängig von der Zeit angegeben. Die durch gefüllte Kreise markierte Kurve gibt die durchschnittliche Zunahme der $OD_{620}$ mit Kelzan® wieder, die durch offene Dreiecke markierte Kurve gibt die Veränderung der $OD_{620}$ ohne Kelzan® wieder. Durch offene Vierecke bzw. offene Rauten sind die Kurven gekennzeichnet, die die Veränderungen der $OD_{620}$ in der Medienkontrolle ohne Zellen mit bzw. ohne Kelzan® wiedergeben. Die schwarzen Balken geben den jeweiligen Standardfehler der Messung an.

**Abbildung 2**

**Reporterstammspezifische und Kelzan®-abhängige Zunahme der GFP-Fluoreszenz in 384-well-MTPs.** Angegeben ist die zeitabhängige Veränderung der relativen Fluoreszenz beruhend auf der Expression des Reportergenprodukts GFP in 384 well MTPs. Die durch Kästen und gefüllte Dreiecke markierten Kurven geben die relative Fluoreszenz im Stamm UMA3 mit bzw. ohne Kelzan® dar. Die durch Rauten bzw. gefüllte Kreise markierten Kurven geben die relative Fluoreszenz im Stamm Um521 mit und ohne Kelzan® wieder. Angegeben ist weiterhin jeweils der Standardfehler der Messreihen.

**Abbildung 3**

**Wachstumstest von *U. maydis* Stämmen in 384 well MTPs. A:** Die Kinetik der Absorption von *U. maydis* Wildtyp-Stämmen wurde bei OD 620 in einer 384 well MTP mit einem Tecan ultra Reader bestimmt (Rhomben). Als Kontrolle diente ein Stamm, dem Hygromycin B in einer Konzentration von 200 μm/ml zugegeben wurde (Kreuze). Die Ausgangs-OD 620 betrug in beiden Stämmen 0,18. Der Stamm ohne Hygromycin zeigte bis 20 h nach dem Start eine OD-Zunahme, während sich beim gleichen Stamm in Gegenwart von Hygromycin eine kontinuierliche Abnahme der optischen Dichte zeigte. **B**: Kinetik der GFP- Expression in transgenen *U. maydis* Stämmen in 384 well MTPs. Die GFP-Fluoreszenz eines *U. maydis* Stammes, in dem die GFP-Expression durch einen starken konstitutiven OMA-Promotor kontrolliert wird (Quadrate), wird in einem Tecan plus Reader bestimmt. Die Anregungswellenlänge betrug 485 nm, dier Emissionswellenlänge 535 nm. Als Kontrolle diente ein Wildtyp-Stamm, der kein GFP exprimiert (Dreiecke). Mit der Zeit zeigt sich beim transgenen Stamm eine Zunahme der GFP-Fluoreszenz mit einem Maximum nach 50 h, während der Wildtyp-Stamm keine Fluoreszenz zeigt. Verglichen mit niedrigeren Temperaturen, erfolgte die OD260-Zunahme und die Zunahme der Fluoreszenz schneller. Versuchsbedingungen: die *U. maydis* Stämme wurden wie zuvor in den Beispielen beschrieben behandelt. Die Lösungen enthielten 1 x Minimalmedium, 0,1 % Kelzan und der Versuch wurde bei 30°C durchgeführt. Alle Experimente wurden unabhängig voneinander wiederholt.

**Literatur**

1. Holliday,R. (1974) Ustilago maydis. In King, R.C. (ed) Handbook of Genetics. Vol.1, Plenum, New York, pp. 575-595.
2. Shabana, Y. M., Charudattan, R., DeValerio, J. T., Elwakil, M. A. (1997): An evaluation of hydrophilic polymers for formulating the bioherbicide agents Alternaria cassiae and A. eichhomiae. Weed Technology 11, 212-220.
3. Spellig T, Bottin A, Kahmann R (1996): Green fluorescent protein (GFP) as a new vital marker in the phytopathogenic fungus *Ustilago maydis. Mol. Gen. Genet.* **252**, 503-509.

4. Tsukada, T., Carleton, S., Fotherinham, S. and HollomanW.K. (1988): Isolation and characterization of an autonomously replicating sequence from Ustilago maydis. Mol. Cell. Biol. 8, 3703-3709

**Patentansprüche**

**1.** Verfahren zur Erhöhung der Wachstumsrate von Zellen in nicht konstant geschüttelten Flüssigmedien, **dadurch gekennzeichnet, dass** man dem Flüssigmedium ein Xanthan in einer Konzentration von 0,08 bis 0,3 Gew.-% zusetzt.

**2.** Verfahren gemäß Anspruch 1, **dadurch gekennzeichnet, dass** es sich bei den Zellen um Pflanzen-, Säuger- oder Insektenzellen handelt.

**3.** Verfahren gemäß Anspruch 1, **dadurch gekennzeichnet, dass** es sich bei den Zellen um Pilzzellen handelt.

**4.** Verfahren gemäß Anspruch 3, **dadurch gekennzeichnet, dass** es sich bei den Pilzzellen um Zellen von *Ustilago maydis* handelt.

**5.** Verfahren gemäß einem der Ansprüche 1 bis 4, **dadurch gekennzeichnet, dass** man dem Flüssigmedium ein Xanthan in einer Konzentration von 0,08 bis 0,2 Gew.-% zusetzt.

**6.** Verfahren gemäß einem der Ansprüche 1 bis 5, **dadurch gekennzeichnet, dass** es sich bei dem Xanthan um Kelzan handelt.

**7.** Verwendung von Verfahren gemäß einem der Ansprüche 1 bis 6 zur Erhöhung der Wachstumsrate von Zellen in nicht konstant geschüttelten Flüssigmedien.

**8.** Verwendung von Verfahren gemäß einem der Ansprüche 1 bis 6 zur Erhöhung der Genexpression in Zellen, die in nicht konstant geschüttelten Flüssigmedien kultiviert werden.

**Claims**

**1.** Method for increasing the growth rate of cells in non-constantly shaken liquid media, **characterized in that** a xanthan is added to the liquid medium at a concentration of from 0.08 to 0.3% by weight.

**2.** Method according to Claim 1, **characterized in that** the cells are plant cells, mammalian cells or insect cells.

**3.** Method according to Claim 1, **characterized in that** the cells are fungal cells.

**4.** Method according to Claim 3, **characterized in that** the fungal cells are *Ustilago maydis* cells.

**5.** Method according to one of Claims 1 to 4, **characterized in that** a xanthan is added to the liquid medium at a concentration of from 0.08 to 0.2% by weight.

**6.** Method according to one of Claims 1 to 5, **characterized in that** the xanthan is kelzan.

**7.** Use of methods according to one of Claims 1 to 6 for increasing the growth rate of cells in non-constantly shaken liquid media.

**8.** Use of methods according to one of Claims 1 to 6 for increasing gene expression in cells which are cultured in non-constantly shaken liquid media.

**Revendications**

**1.** Procédé pour élever le taux de prolifération de cellules dans des milieux liquides non agités constamment par secousses, **caractérisé en ce qu'**on ajoute au milieu liquide un xanthane à une concentration de 0,08 à 0,3 % en poids.

2. Procédé suivant la revendication 1, **caractérisé en ce que** les cellules sont des cellules de végétaux, de mammifères ou d'insectes.

3. Procédé suivant la revendication 1, **caractérisé en ce que** les cellules sont des cellules de champignons.

4. Procédé suivant la revendication 3, **caractérisé en ce que** les cellules de champignons sont des cellules *d'Ustilago maydis*.

5. Procédé suivant l'une des revendications 1 à 4, **caractérisé en ce qu'**on ajoute au milieu liquide un xanthane à une concentration de 0,08 à 0,2 % en poids.

6. Procédé suivant l'une des revendications 1 à 5, **caractérisé en ce que** le xanthane consiste en kelzane.

7. Utilisation de procédés suivant l'une des revendications 1 à 6 pour élever le taux de prolifération de cellules dans des milieux liquides non agités constamment par secousses.

8. Utilisation de procédés suivant l'une des revendications 1 à 6 pour accroître l'expression génique dans des cellules qui sont cultivées dans des milieux liquides non agités constamment par secousses.

# Fig. 1

Wachstumskurve

# Fig. 2

GFP-Expression

# Fig. 3A

# Fig. 3B